# EUROPÄISCHE PATENTSCHRIFT

(11) **EP 0 921 846 B1**
(45) Veröffentlichungstag und Bekanntmachung des Hinweises auf die Patenterteilung: **13.06.2001**
(21) Anmeldenummer: 97937509.4
(22) Anmeldetag: 19.07.1997
(51) Int. Cl.: B01D 8/00, B01D 5/00, A61M 16/10

(54) **VERFAHREN ZUR ONLINE-RÜCKGEWINNUNG VON XENON AUS NARKOSEGAS**
ON-LINE RECOVERY OF XENON FROM ANAESTHETIC GAS
PROCEDE DE RECUPERATION DIRECTE DE XENON A PARTIR D'UN GAZ ANESTHESIANT

(30) Priorität: 30.08.1996 DE 19635002
(43) Veröffentlichungstag der Anmeldung: 16.06.1999
(73) Patentinhaber: MESSER GRIESHEIM GMBH, 60547 Frankfurt (DE)
(72) Erfinder: ESCHWEY, Manfred, D-40489 Düsseldorf (DE); HAMM, Reiner, D-47506 Neukirchen-Vluyn (DE); NEU, Peter, D-45475 Mülheim (DE); SCHMIDT, Renate, D-47249 Duisburg (DE); SCHROEDER, Georg, D-44879 Bochum (DE)
(86) Internationale Anmeldenummer: EP9703885
(87) Internationale Veröffentlichungsnummer: WO9808583

(56) Entgegenhaltungen:
- DE-C- 4 411 533
- US-A- 3 853 507
- US-A- 4 668 261

## Beschreibung

Die Erfindung betrifft ein Verfahren zum Abtrennen von Xenon aus einem bei einer Narkose anfallenden Gasgemisch, ein Verfahren zum Recyclen eines Narkosegases, welches als Komponenten Xenon und ein oder mehrere Verbrauchsgase enthält, ein Narkosegerät mit einer Vorrichtung zum Rückgewinnen von Xenon aus einem bei einer Narkose anfallenden Gasgemisch und eine entsprechende Verwendung der Vorrichtung in einem Narkosegerät.

Die narkotischen Wirkungen von Xenon sind seit den 40er Jahren bekannt. Eine Mischung von etwa 80% Xenon und 20% Sauerstoff wird als nahezu ideales Narkosegas betrachtet, das gegenüber den heute überwiegend verwendeten Narkosegasen auf der Basis von Lachgas zahlreiche Vorteile hat. Wegen der hohen Kosten von Xenon werden Xenon-Narkosen im klinischen Bereich praktisch nicht angewandt. Um diese Kosten zu verringern, wurden daher z.B. in DE 44 11 533 C1 Narkosegeräte mit einer Rückgewinnungsanlage für Xenon vorgeschlagen. Bei der in DE 44 11 533 C1 vorgeschlagenen Rückgewinnungsanlage wird das ausgeatmete Atemgas nach einer Vorreinigung komprimiert und in einen Druckbehälter eingeleitet, welcher in eine Kühlvorrichtung aufgenommen ist. Über die Kühlvorrichtung wird der Druckbehälter so weit abgekühlt, daß das rückzugewinnende Xenon sich verflüssigt. Die gasförmigen Bestandteile in dem Druckbehälter werden über eine Abströmdrossel abgelassen. Wenn eine genügende Menge an flüssigem Xenon in dem Druckbehälter vorhanden ist, wird dieses in einen weiteren Behälter umgepumpt. Diese Vorrichtung hat den Nachteil eines hohen apparativen Aufwands für das Komprimieren des Gases und das Abkühlen eines ganzen Behälters. Außerdem ist der Transfergrad der Xenonrückgewinnung nicht zufriedenstellend.

Aus DE 35 18 283 A1 ist ein Verfahren zum Entfernen leicht flüchtiger Verunreinigungen aus in der Halbleitertechnik anfallenden Gasen bekannt, bei dem das zu reinigende Gas in einem Vakuumsystem auf eine Kaltfläche eines Kondensators geleitet wird, an welchem sich das zu reinigende Gas niederschlägt, während die leichter flüchtigen Verunreinigungen kontinuierlich gasförmig aus dem Vakuumsystem abgezogen werden. Auch bei diesem Verfahren ist der apparative Aufwand zur Rückgewinnung aufgrund des verwendeten Vakuumsystems hoch.

In US-A-4 668 261 wird die Entfernung einer Gaskomponente (Xenon) aus einem Gasgemisch durch Ausfrieren in einer Kühlfalle beschrieben. Bei der eingesetzten Kühlfalle mit Gegenstromkühlung wird die Temperatur der Kühlflächen durch entsprechende Kältemittelzufuhr und Heizung geregelt.

Es ist die Aufgabe der Erfindung, ein einfaches und wirtschaftliches Verfahren zum Abtrennen von Xenon aus dem exspiratorischen Atemgas eines Patienten bei der Xenon-Narkose und eine zugehörige Vorrichtung zu schaffen. Eine weitere Aufgabe der Erfindung besteht darin, einen wirtschaftlichen Recyclingprozeß für ein Narkosegas auf Xenonbasis zu schaffen.

Diese Aufgaben werden durch ein Verfahren mit den in Anspruch 1, 2 oder 3 beschriebenen Merkmalen gelöst.

Überraschenderweise hat sich herausgestellt, daß eine Abtrennung von Xenon mit einer akzeptablen Reinheit auch noch bei einer Kondensation unter einem Druck im Bereich von 0,6 bar bis zu 150 bar möglich ist. Bei Verwendung des erfindungsgemäßen Recyclingverfahrens für ein Narkosegas können die Anforderungen an die Reinheit des Xenons sogar noch herabgesetzt werden, da sich Restanteile von Sauerstoff in dem rückgewonnenen Xenon durch einen entsprechend geringeren Anteil des Sauerstoffs beim Neumischen des Narkosegases kompensieren lassen.

Vorteilhafterweise wird durch die Verwendung eines Wärmetauschers, der aus einem Rohr oder einem Rohrbündel besteht, der Aufwand für das Auskondensieren des Xenons verringert, da nun nicht mehr der ganze Behälter, sondern eine vergleichsweise geringe Kondensationsfläche auf die für das Kondensieren des Xenons in fester Form erforderliche Temperatur abgekühlt werden muß. Gleichzeitig eröffnet dies eine Möglichkeit, die Schichtdicke zu kontrollieren. Da das an dem Wärmetauscher abgeschiedene feste Xenon isolierend wirkt, wird das Innere des Behälters durch den Wärmetauscher mit wachsender Dicke der festen Xenonschicht weniger stark gekühlt, so daß aus der Temperatur im Behälter auf die Dicke der Xenonschicht auf dem Wärmetauscher geschlossen werden kann.

Der beim Verdampfen des abgeschiedenen Xenons entstehende Druck kann erfindungsgemäß zum Überleiten des rückgewonnenen Xenons in einen anderen Behälter verwendet werden. Pumpen für die Förderung des rückgewonnenen Xenons zur Weiterverabeitung können daher entfallen oder kleiner dimensioniert werden.

Das erfindungsgemäße Verfahren läßt sich als Durchlaufverfahren bei niedrigem Druck konzipieren, bei dem vorgereinigtes Atemgas entlang eines Rohrbündel-Wärmetauschers strömt. Vorteilhafterweise sind die Rohrbündel dieses Wärmetauschers so gestaltet, daß sich ein möglichst langer Strömungsweg entlang der gekühlten Rohre ergibt. Der Druck des die Abscheideeinrichtung durchströmenden Gases liegt dabei typischerweise im Bereich von 0,6 bar bis 5 bar, vorzugsweise in der Nähe des Atmosphärendrucks.

Erfindungsgemäß kann das Atemgas auch in einem Druckbehälter gespeichert werden, in dem nach dem Einströmen einer ausreichenden Gasmenge das Xenon in fester Form auf einem Wärmetauscher auskondensiert wird und die nicht kondensierten Gase als Kopfgas abgelassen werden. Beim anschließenden Verdampfen des in fester Form abgeschiedenen Xenons in dem verschlossenen Behälter ergibt sich ein Druck, welcher zum einen das Ableiten des abgetrennten Xenons in einen weiteren Behälter ohne eine zusätzliche Pumpe ermöglicht und andererseits auch in einem Bereich liegt, der dem Druck kommerzieller Xenon-Gasflaschen entspricht. Ist zum Beispiel der Abscheidebehälter auf einen Druck von 150 bar ausgelegt, ergibt sich für das abgeschiedene Xenon nach dem Verdampfen ein Druck von 65 bar. Der Abscheidebehälter kann daher unmittelbar als Xenonspeicher für die weitere Verarbeitung verwendet werden.

Gemäß einer vorteilhaften Ausführungsform der Erfindung werden zwei Abscheideeinrichtungen im Tandem-Verfahren verwendet. Dies ermöglicht es, in einer der beiden Abscheideeinrichtungen das exspiratorische Atemgas zu sammeln bzw. Xenon auszukondensieren, während in der anderen Abscheideeinrichtung bereits auskondensiertes Xenon verdampft wird.

Weitere Merkmale und Vorteile der Erfindung werden aus der folgenden detaillierten Beschreibung zweier Ausführungsbeispiele der Erfindung anhand der beigefügten Zeichnungen deutlich.
- Fig. 1: zeigt schematisch eine Vorrichtung zur Xenon-Rückgewinnung aus Narkosegas nach einem Niederdruckverfahren,
- Fig. 2: zeigt schematisch eine Vorrichtung zur Xenon-Rückgewinnung nach einem Hochdruckverfahren.

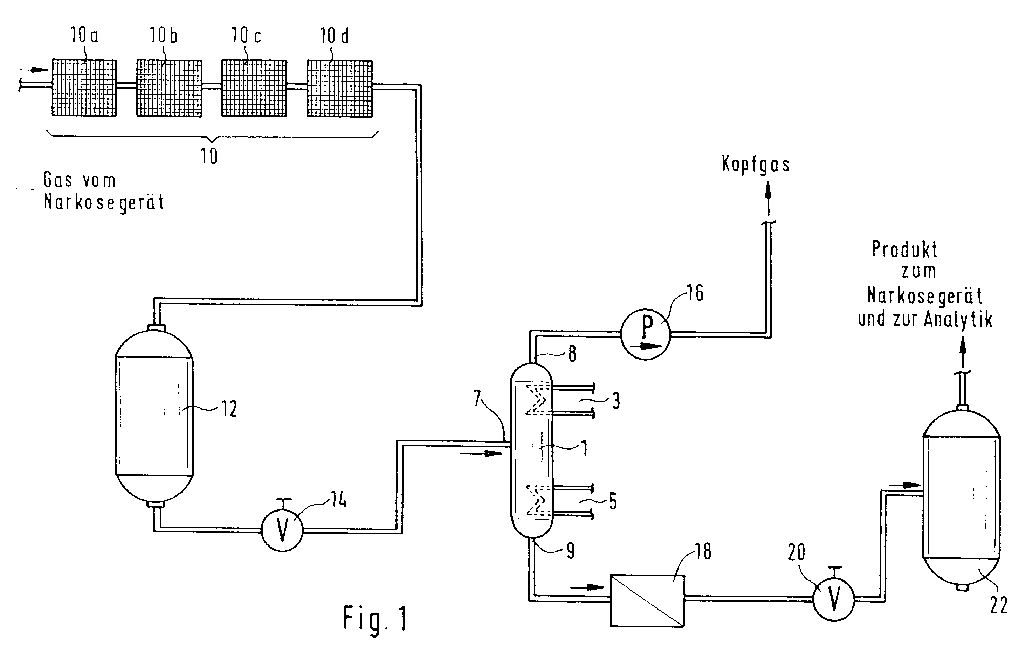

In Fig. 1 ist eine erste Ausführungsform der erfindungsgemäßen Xenon-Rückgewinnungsanlage dargestellt, bei der das Xenon mit einem Niederdruckverfahren abgetrennt wird. Kernstück der Anlage ist eine Abscheideeinrichtung 1, welche einen durch flüssigen Stickstoff gekühlten Wärmetauscher 3 und eine Heizung 5 aufweist, die in der Zeichnung nur in Form von funktionellen Symbolen angedeutet sind. Die Abscheideeinrichtung 7 weist einen Einlaß 7 für exspiratorisches Atemgas von einem Narkosegerät (nicht dargestellt) sowie zwei Auslässe 8 und 9 auf. Der Wärmetauscher 3 besteht aus einem Rohrbündel, durch welches flüssiger Stickstoff gebildet wird. Er könnte jedoch auch durch eine thermisch gut leitende Oberfläche eines Reservoirs für flüssigen Stickstoff geleitet werden. Der Wärmetauscher 3 befindet sich im Strömungsweg von dem Einlaß 7 zu dem Auslaß 8 und ist in üblicher Weise so gestaltet, daß eine möglichst große Wärmetauscherfläche in Kontakt mit der Gasströmung kommt. Die Heizung 5 ist eine Elektroheizung, welche den Behälter der Abscheideeinrichtung 1 erwärmt.

Zwischen dem Narkosegerät und dem Einlaß 7 ist eine Vorreinigungseinrichtung 10 mit vier Vorreinigungsstufen 10a bis 10d zwischengeschaltet. Die vier Vorreinigungsstufen dienen dem Entfernen von verschiedenen Verunreinigungen, die in dem exspiratorischen Atemgas von dem Narkosegerät enthalten sind. So kann die Reinigungsstufe 10a Natronkalk zum Entfernen von Kohlendioxid, die Reinigungsstufe 10b ein Molsieb zum Entfernen von Feuchtigkeit in dem Atemgas, die Reinigungsstufe 10c einen Aktivkohlefilter zum Entfernen von Kohlenwasserstoffen, die als Stoffwechselprodukte in dem Atemgas enthalten sind, und die Reinigungsstufe 10d einen Partikelfilter, z.B. einen HEPA-Filter, zum Entfernen von Schwebeteilchen, Keimen und dergleichen enthalten. Die Vorreinigungseinrichtung 10 ist über einen Zwischenspeicher 12 und ein Absperrventil 14 mit dem Eingang 7 verbunden.

Der erste Auslaß 8 der Abscheideeinrichtung 1 ist mit einer Membranpumpe 16 verbunden, die das von ihr angesaugte Gas entweder in die freie Atmosphäre oder in einen Abgassammelbehälter (nicht dargestellt) ableitet. Der zweite Ausgang ist über eine Rückflußsperre 18 und ein Absperrventil 20 mit einem Sammelbehälter 22 für das rückgewonnene Xenon verbunden.

Die in Fig. 1 dargestellte Anlage arbeitet wie folgt: das von dem Narkosegerät kommende Atemgas wird zunächst durch die Vorreinigungseinrichtung 10 geleitet, in der Verunreinigungen, wie Kohlenwasserstoffe und Keime, sowie Substanzen mit einem höheren Gefrierpunkt als Xenon (H₂O, CO₂) aus dem Gas entfernt werden. Das vorgereinigte Gas wird dann in dem Zwischenspeicher 12 gespeichert. Wenn die Abscheideeinrichtung 1 zum Aufnehmen von Atemgas bereit ist und/oder eine ausreichende Menge an Gas in dem Zwischenbehälter 12 gespeichert ist, wird das Absperrventil 14 geöffnet und das Gas strömt über den Einlaß 7 in die Abscheideeinrichtung 1. Dabei wird durch die Pumpe 16 eine Strömung über den Wärmetauschern 3 erzeugt, dessen Oberfläche aufgrund der Kühlung durch flüssigen Stickstoff eine Temperatur von ungefähr -196°C hat. Auf der Wärmetauscherfläche des Wärmetauschers 3 scheidet sich das Xenon (Gefrierpunkt: -112°C) in fester Form ab, während die Hauptverunreinigungen, nämlich Sauerstoff (Gefrierpunkt: -219°C) und Stickstoff (Gefrierpunkt: -210°C) gasförmig bleiben und von der Pumpe 16 als Kopfgas abgesaugt werden. Da die Komponenten mit einem höheren Gefrierpunkt als dem von Xenon in der Vorreinigungseinrichtung 10 abgetrennt worden sind, enthält das an dem Wärmetauscher abgeschiedene Xenon nur noch sehr geringe Anteile von Verunreinigungen.

Der vorangehend beschriebene Abscheideprozeß wird fortgesetzt, bis eine ausreichende Schichtdicke des Xenon auf dem Wärmetauscher 3 erreicht ist. Das Vorliegen einer ausreichenden Schichtdicke kann z.B. durch ein Durchflußmeßgerät in der Leitung zu dem Einlaß 7 festgestellt werden, da einer bestimmten Durchflußmenge eine bestimmte Schichtdicke von Xenon auf dem Wärmetauscher entspricht. Eine andere Möglichkeit besteht darin, über einen Temperaturfühler im Behälter der Abscheideeinrichtung zu detektieren, wie stark die Temperatur in dem Behälter gegenüber dem Beginn des Abscheideprozesses angestiegen ist. Da das abgeschiedene Xenon den Wärmetauscher 3 isoliert, entspricht einer bestimmten Schichtdicke ein gewisser Temperaturanstieg in dem Behälter der Abscheideeinrichtung 1. Wenn die gewünschte Schichtdicke erreicht ist, wird die Zufuhr von Atemgas von dem Zwischenspeicher 12 mit Hilfe des Ventils 14 unterbrochen und der Ausgang 8 der Abscheideeinrichtung wird verschlossen. Anschließend wird der Behälter der Abscheideeinrichtung 1 mit Hilfe der Heizung 5 erwärmt, so daß das an dem Wärmetauscher 3 abgeschiedene Xenon verdampft wird. Dadurch baut sich ein Druck in dem Behälter der Abscheideeinrichtung 1 auf. Nach dem Verdampfen des Xenons werden der Ausgang 9 und das Ventil 20 geöffnet, so daß das in der Abscheideeinrichtung 1 befindliche gasförmige Xenon aufgrund des aufgebauten Drucks über die Rückflußsperre 18 und das Ventil 20 in den Xenonbehälter 22 strömt.

Die vorangehend beschriebene Ausführungsform kann in verschiedener Hinsicht abgewandelt werden. So kann die Verbindung zwischen dem Narkosegerät und dem Zwischenspeicher 12 so gestaltet sein, daß sich bei geschlossenem Ventil 14 in dem Zwischenspeicher ein moderater Druck, etwa im Bereich von 3 bis 5 bar, einstellt. In diesem Fall kann auf die Pumpe 16 verzichtet werden und der Ausgang 8 direkt mit der Atmosphäre verbunden werden. Das in dem Zwischenspeicher 12 enthaltene Gas fließt dann nach dem Öffnen des Ventils 14 aufgrund des Druckgefälles gegenüber der Atmosphäre über den Einlaß 7 und den Auslaß 8 durch die Abscheideeinrichtung 1 und den Wärmetauscher 3.

Weiterhin kann die Heizung so betrieben werden, daß nach dem Verschließen des Einlasses 7 das Xenon zunächst nur verflüssigt wird, so daß in dem festen Xenon eingeschlossene Verunreinigungen freigesetzt und über die Pumpe 16 als Kopfgas abgesaugt werden.

Es ist auch möglich, das rückgewonnene Xenon in flüssiger Form dem Xenonspeicher 22 zuzuführen. In diesem Fall wird das abgeschiedene Xenon nur verflüssigt und in der Verbindung zwischen dem Ausgang 9 und dem Behälter 22 eine Flüssigkeitspumpe vorgesehen.

Schließlich kann der Behälter der Abscheideeinrichtung auch nur mit einem Auslaß 8 versehen sein, durch den sowohl das die Verunreinigungen enthaltende Gas beim Abscheideprozeß als auch später das rückgewonnene Xenon abgeführt werden können, wobei ein Mehrwegventil das austretende Gas wahlweise in die Atmosphäre oder zu dem Xenonspeicher 22 leitet.

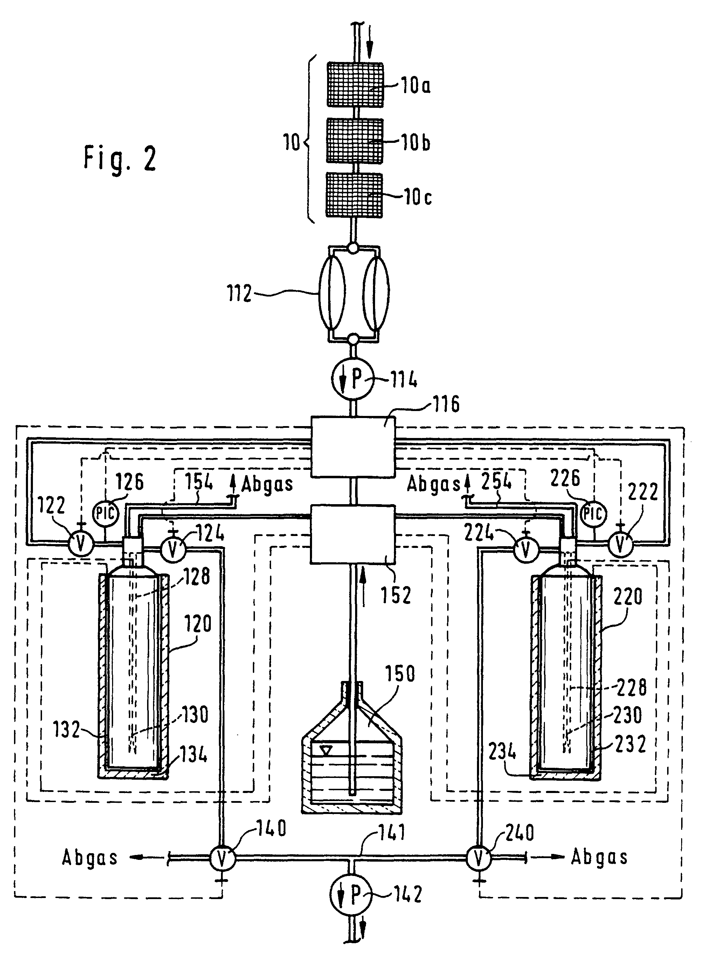

Fig. 2 zeigt eine Anlage zur Xenonrückgewinnung aus Narkosegas in einem Hochdruckverfahren, wobei Gasleitungen mit durchgezogenen Linien und Meß- und Steuerleitungen (Temperatur, Druck, Pneumatik, Strom) mit gestrichelten Linien dargestellt sind. Diese Anlage weist wieder eine Vorreinigungseinrichtung 10 mit Reinigungsstufen 10a bis 10c zur Reinigung des von dem Narkosegerät kommenden exspiratorischen Atemgases auf. Die in der Vorreinigungseinrichtung 10 enthaltenen Reinigungsstufen sind nur beispielhaft angegeben; selbstverständlich können auch hier ein HEPA-Filter und/oder weitere Reinigungsstufen eingesetzt werden. Die Vorreinigungseinrichtung 10 ist über eine druckfreie Pumpensteuerung 112 bzw. Pufferung mit einer Pumpe 114 verbunden, welche das vorgereinigte Atemgas über eine Strangumschaltung 116 wahlweise zu einem von zwei Abscheidebehältern 120, 220 leitet, die identisch aufgebaut sind. Die nachfolgende Erläuterung ihres Aufbaus bezieht sich auf die erste Abscheideeinrichtung, wobei die Bezugsziffern für die zweite Abscheideeinrichtung in Klammern angegeben sind. Jede der beiden Abscheideeinrichtungen besteht aus einem druckfesten Behälter, der für einen Druck von 150 bar ausgelegt ist und ein Einlaßventil 122 (222) und ein Auslaßventil 124 (224) aufweist. Ein Drucksensor 126 (226) erfaßt den Druck im Behälter. In dem Behälter befindet sich ein Wärmetauscher in Form eines Kühlrohrs 128 (228) aus Edelstahl, das von flüssigem Stickstoff durchströmt werden kann. Ein Temperaturmeßfühler 130 (230) erfaßt die Temperatur im Innenraum des Behälters, während ein Temperaturmeßfühler 132 (232) die Temperatur an der Behälteraußenwand erfaßt. Der Behälter selbst ist von einer Wärmeisolierung 134 (234) umgeben. Das Einlaßventil 122 (222) ist über die Strangumschaltung 116 mit der Pumpe 114 verbunden, während das Auslaßventil 124 (224) mit einem Mehrwegventil 140 (240) verbunden ist, das ebenfalls von der Strangumschaltung 116 angesteuert wird. Das Mehrwegventil 140 (240) leitet das von dem Auslaßventil 124 kommende Gas je nach Ansteuerung entweder als Abgas ab oder über eine Produktleitung 141 zu einer Pumpe 142. Das Kühlrohr 128 (228) ist mit einer Versorgung für flüssigen Stickstoff 150 verbunden, wobei eine Temperatursteuerung 152 in Abhängigkeit von den Meßwerten der Temperaturfühler 130 (230) und 132 (232) die Menge des flüssigen Stickstoffs bestimmt, welche durch das Kühlrohr strömt. Der dabei verdampfte flüssige Stickstoff wird als Abgas über die Leitung 154 (254) abgeführt.

Die in Fig. 2 dargestellt Anlage arbeitet wie folgt. Das in der Vorreinigungseinrichtung 10 vorgereinigte exspiratorische Atemgas von dem Narkosegerät wird über die Pumpensteuerung und die Pumpe 114 zunächst ausschließlich zu einer der beiden Abscheideeinrichtungen, z.B. Einrichtung 120, geleitet, wobei das Auslaßventil 124 dieser Einrichtung geschlossen bleibt. Wenn der Enddruck von 150 bar in dem Behälter der Abscheideeinrichtung 120 erreicht ist, schließt die Strangumschaltung 116 das Einlaßventil 122 und leitet alles weitere von der Pumpe 114 kommende Atemgas zu der anderen Abscheideeinrichtung 220. In der Zwischenzeit wird von dem Stickstoffreservoir 150 durch die Temperatursteuerung 152 flüssiger Stickstoff in das Kühlrohr 128 eingeleitet, so daß sich festes Xenon auf dem Kühlrohr 128 abscheidet. Wenn das im Gasgemisch befindliche Xenon im wesentlichen abgeschieden ist, was z.B. durch Abmessen einer charakteristischen Zeitdauer oder durch Messen des Temperaturverlaufs in dem Behälter der Abscheideeinrichtung 120 festgestellt werden kann, wird das zugehörige Mehrwegventil 140 so eingestellt, daß das von dem Auslaßventil 124 kommende Gas als Abgas abgeführt wird, und das Auslaßventil 124 wird geöffnet. Das in dem Behälter befindliche Kopfgas, welches die nicht kondensierten Komponenten des eingeleiteten Gasgemischs enthält, wird abgelassen und als Abgas abgeführt. Danach wird das Auslaßventil 124 geschlossen und der Behälter durch eine Heizung (nicht dargestellt) erwärmt, so daß das an dem Kühlrohr 128 abgeschiedene Xenon verdampft wird. Dabei stellt sich innerhalb des Behälters typischerweise ein Druck von 65 bar ein, der dem Druck von handelsüblichen Xenonflaschen entspricht. Nach Umstellen des Mehrwegventils 140 wird dann das so gewonnene gasförmige Xenon über das Auslaßventil 124 und die Produktleitung 141 zu der Pumpe 142 geleitet, welche das Xenon zu einer nachgeschalteten Analyse- oder Verarbeitungsstufe leitet. Danach ist die Abscheideeinrichtung 120 wieder bereit zum Aufnehmen von exspiratorischem Atemgas von der Pumpe 114. Sobald in der zweiten Abscheideeinrichtung 220, in welche alles in der Zwischenzeit angefallene exspiratorische Atemgas gepumpt worden ist, der Maximaldruck von 150 bar erreicht ist, wird das zugehörige Einlaßventil 222 geschlossen und die Pumpe 114 mit Hilfe der Strangumschaltung 116 wieder mit der ersten Abscheideeinrichtung 120 verbunden. Die beiden Abscheideeinrichtungen 120 und 220 arbeiten also im Tandemverfahren, so daß eine kontinuierliche Verarbeitung des anfallenden exspiratorischen Atemgases erfolgt.

Auch bei dieser zweiten Ausführungsform der erfindungsgemäßen Rückgewinnungsanlage können Abwandlungen ähnlich wie bei der ersten vorangehend beschriebenen Ausführungsform vorgesehen sein. So kann z.B. auch hier das Kühlrohr 128 als Rohrbündel ausgebildet sein. Die Produktpumpe 142 kann, je nach der Art der Weiterverarbeitung, entfallen. Da der Druck des verdampften Xenons (typischerweise 65 bar) dem Druck von handelsüblichen Xenonflaschen entspricht, können die Behälter der Abscheideeinrichtungen 120 bzw. 220 auch direkt als Druckgasflaschen verwendet werden. In diesem Fall kann vorgesehen sein, daß die Behälter nach jedem Rückgewinnungsvorgang ausgetauscht werden. Selbstverständlich ist es auch möglich, mehr als zwei Behälter einzusetzen, welche dann nacheinander von der Strangumschaltung 116 gefüllt bzw. zur Rückgewinnung angesteuert werden. Schließlich ist es auch möglich, zwei Niedrigdruck-Anlagen, wie in Fig. 1 dargestellt mit Hilfe einer Strangumschaltung zu koppeln oder eine Niedrigdruck-Anlage mit einer Hochdruck-Anlage über eine Strangumschaltung zu koppeln, so daß je nach Bedarf und Gegebenheiten nach dem Niedrigdruckverfahren oder dem Hochdruckverfahren gearbeitet werden kann. Andererseits ist es auch möglich, eine einzige Hochdruck-Abscheideeinrichtung, z.B. die in Fig. 2 mit 120 bezeichnete Abscheideeinrichtung, zu verwenden, wobei analog zur Ausführungsform der Fig. 1 ein Zwischenspeicher vorgesehen wird, der das Atemgas aufnimmt, das während des Verdampfens des auskondensierten Xenons anfüllt.

Da das rückgewonnene Xenon wieder als Narkosegas verwendet wird, braucht das rückgewonnene Xenon nicht hochrein zu sein. Erfindungsgemäß wird das rückgewonnene Xenon vor dem Neumischen des Narkosegases analysiert und bei Vorliegen von Restkonzentrationen an Sauerstoff und Stickstoff wird der entsprechende Anteil von Sauerstoff bzw. Stickstoff, der dem Xenon beim Neumischen des Narkosegases zugesetzt wird, entsprechend vermindert. Sonstige etwaige Restkonzentrationen von Verunreinigungen, z.B. CO₂, sind physiologisch unbedenklich und können im übrigen nach einer Analyse des rückgewonnenen Xenons bei der Dosierung der Narkose entsprechend berücksichtigt werden.

## Patentansprüche

1. Verfahren zum Abtrennen von Xenon aus einem bei einer Narkose anfallenden Gasgemisch, welches die folgenden Schritte umfaßt:
a) In-Kontakt-Bringen des Gasgemisches mit einer Kühlfläche, die sich auf einer Temperatur unterhalb des Schmelzpunktes von Xenon befindet, bei einem Druck von 0,6 bis 150 bar,
b) Ableiten der nicht an der Kühlfläche in festem Zustand kondensierten Anteile des Gasgemisches,
c) Erwärmen der an der Kühlfläche kondensierten Komponente über den Schmelzpunkt von Xenon hinaus, wobei der Schritt des Erwärmens des an der Kühlfläche kondensierten Xenons das Verflüssigen dieser Komponente umfaßt.

2. Verfahren zum Abtrennen von Xenon aus einem bei einer Narkose anfallenden Gasgemisch, welches die folgenden Schritte umfaßt:
a) In-Kontakt-Bringen des Gasgemisches mit einer Kühlfläche, die sich auf einer Temperatur unterhalb des Schmelzpunktes von Xenon befindet, bei einem Druck von 0,6 bis 150 bar,
b) Ableiten der nicht an der Kühlfläche in festem Zustand kondensierten Anteile des Gasgemisches,
c) Erwärmen der an der Kühlfläche kondensierten Komponente über den Schmelzpunkt von Xenon hinaus
und die Abtrennung mittels zwei oder mehr parallelen Abscheideeinrichtungen, wobei während des Schritts des Erwärmens des kondensierten Xenons in einer Abscheideeinrichtung das anfallende Gasgemisch zumindest zu einer weiteren Abscheideeinrichtung zum Auskondensieren des abzutrennenden Xenons geleitet wird.

3. Verfahren zum Recyclen eines Narkosegases, welches als Komponenten Xenon und ein oder mehrere Verbrauchsgase enthält, welches die folgenden Schritte umfaßt:
a) Abtrennen des rückzugewinnenden Xenons aus dem verunreinigten Gasgemisch mit einem Verfahren zum Abtrennen von Xenon aus einem Gasgemisch, welches die folgenden Schritte umfaßt:
a.a) In-Kontakt-Bringen des Gasgemisches mit einer Kühlfläche, die sich auf einer Temperatur unterhalb des Schmelzpunktes von Xenon befindet, bei einem Druck von 0,6 bis 150 bar,
a.b) Ableiten der nicht an der Kühlfläche in festem Zustand kondensierten Anteile des Gasgemisches,
a.c) Erwärmen der an der Kühlfläche kondensierten Komponente über den Schmelzpunkt von Xenon hinaus, wobei die Verbrauchsgase nach dem Auskondensieren des rückzugewinnenden Xenons zusammen mit den Verunreinigungen abgeführt werden,
b) Bestimmen des Restanteils der Verbrauchsgase in dem rückgewonnenen Gas,
c) Neumischen des Gasgemischs aus dem rückgewonnenen Gas unter Zusetzen eines Anteils von frischen Verbrauchsgasen wobei die Menge der zugesetzten Verbrauchsgase in Abhängigkeit von der Konzentration der Verbrauchsgase in den rückgewonnenen Gasen bestimmt wird.

4. Verfahren nach Anspruch 3,
dadurch gekennzeichnet,
daß Sauerstoff ein Verbrauchsgas ist.

5. Verwendung einer Vorrichtung, welche umfaßt:
- eine Abscheideeinrichtung (1; 120, 220) mit einem Raum zum Einleiten eines Gasgemischs, der eine Kühlfläche (3; 128, 228) aufweist,
- eine Einrichtung zum Abkühlen der Kühlfläche auf eine Temperatur unterhalb des Schmelzpunktes von Xenon,
- eine Heizeinrichtung (5) zum Erwärmen von festen Komponenten, die an der Kühleinrichtung kondensiert sind,
als Bestandteil eines Narkosegerätes zum Abtrennen von Xenon aus einem bei einer Narkose anfallenden Gasgemisch.

6. Verwendung nach Anspruch 5,
gekennzeichnet durch eine Vorrichtung mit einer Einrichtung zum Durchleiten des Gasgemischs durch die Abscheideeinrichtung (1) von einem Gaseinlaß (7) über einen Innenraum mit der Kühlfläche (3) zu einem Gasauslaß (8), wobei die Kühlfläche (3) im Strömungsweg des Gases liegt.

7. Verwendung nach Anspruch 5 oder 6,
dadurch gekennzeichnet,
daß die Abscheideeinrichtung der Vorrichtung einen verschließbaren Behälter aufweist, in dem sich die Kühlfläche (128) und die Heizeinrichtung befinden.

8. Verwendung nach Anspruch 5 bis 7,
gekennzeichnet durch
eine Vorrichtung mit einer Pumpe (114) zum Einspeisen des Gasgemisches in den Behälter unter Druck und eine Einrichtung (124) zum Ablassen von Kopfgas aus dem Behälter.

9. Verwendung nach Anspruch 5 bis 8,
gekennzeichnet durch
eine Vorrichtung mit zwei oder mehr Abscheideeinrichtungen (120, 220) und eine Umschalteinrichtung (116) zum wahlweisen Einleiten des Gasgemischs in eine der Abscheideeinrichtungen (120, 220).

10. Narkosegerät mit a) einer Vorrichtung zum Rückgewinnen von Xenon aus einem bei der Narkose anfallenden Gas mit einer Abscheideeinrichtung (1; 120, 220) mit einem Raum zum Einleiten eines Gasgemischs, der eine Kühlfläche (3; 128, 228) aufweist, eine Einrichtung zum Abkühlen der Kühlfläche auf eine Temperatur unterhalb des Schmelzpunktes von Xenon, eine Heizeinrichtung (5) zum Erwärmen von festen Komponenten, die an der Kühleinrichtung kondensiert sind,
b) mit einer Analyseeinrichtung zum Analysieren des rückgewonnenen Xenon und
c) mit einer Mischeinrichtung zum Neumischen eines Narkosegases unter Verwendung von rückgewonnenem Xenon, welche das Mischungsverhältnis in Abhängigkeit von dem Analyseergebnis der Analyseeinrichtung einstellt.

## Claims

1. Process for separating off xenon from a gas mixture produced in anaesthesia, which comprises the following steps:
a) bringing the gas mixture into contact with a cooling surface which is at a temperature below the melting point of xenon, at a pressure of 0.6 to 150 bar,
b) leading off those components of the gas mixture which are not condensed in solid state on the cooling surface, and
c) heating the component which has condensed on the cooling surface to above the melting point of xenon, the step of heating the xenon condensed on the cooling surface comprising the liquefaction of this component.

2. Process for separating off xenon from a gas mixture produced in anaesthesia, which comprises the following steps:
a) bringing the gas mixture into contact with a cooling surface which is at a temperature below the melting point of xenon, at a pressure of 0.6 to 150 bar,
b) leading off those components of the gas mixture which are not condensed in solid state on the cooling surface, and
c) heating the component which has condensed on the cooling surface to above the melting point of xenon, and the separation by means of two or more parallel deposition apparatuses, in which process, during the step of heating the condensed xenon in one deposition apparatus, the gas mixture produced is passed at least to one further deposition apparatus for condensing out the xenon to be separated off.

3. Process for recycling an anaesthetic gas whose components comprise xenon and one or more disposable gases, which comprises the following steps:
a) separating off the xenon to be recovered from the contaminated gas mixture by a process for separating off xenon from a gas mixture, which comprises following steps:
a.a) bringing the gas mixture into contact with a cooling surface which is at a temperature below the melting point of xenon, at a pressure of 0.6 to 150 bar,
a.b) leading off those components of the gas mixture which are not condensed in solid state on the cooling surface, and
a.c) heating the component which has condensed on the cooling surface to above the melting point of xenon, the disposable gases being led off, following the condensation of the xenon to be recovered, together with the impurities,
b) determining the residual content of the disposable gases in the recovered gas,
c) remixing the gas mixture from the recovered gas while adding a proportion of fresh disposable gas, the amount of disposable gases added being determined as a function of the concentration of the disposable gases in the recovered gases.

4. Process according to Claim 3, characterized in that oxygen is a disposable gas.

5. Use of a device, which comprises:
- a deposition apparatus (1; 120, 220) having a chamber for passing in a gas mixture, which chamber has a cooling surface (3; 128, 228),
- an apparatus for cooling the cooling surface to a temperature below the melting point of xenon, and
- a heater (5) for heating solid components which have condensed on the cooling device as part of an anaesthetic machine for separating off xenon from a gas mixture obtained in anaesthesia.

6. Use according to Claim 5, characterized by a device having an apparatus for passing the gas mixture through the deposition apparatus (1) from a gas inlet (7) via an interior chamber containing the cooling surface (3) to a gas outlet (8), the cooling surface (3) lying in the flow path of the gas.

7. Use according to Claim 5 or 6, characterized in that the deposition apparatus of the device has a sealable vessel in which the cooling surface (128) and the heater are located.

8. Use according to Claim 5 to 7, characterized by a device having a pump (114) for feeding the gas mixture into the vessel under pressure and a device (124) for letting off overhead gas from the vessel.

9. Use according to Claims 5 to 8, characterized by a device having two or more deposition apparatuses (120, 220) and a switchover device (116) for passing the gas mixture alternatively into one of the deposition apparatuses (120, 220).

10. Anaesthetic machine comprising a) a device for recovering xenon from a gas obtained in anaesthesia having a deposition apparatus (1; 120, 220) with a chamber for the introduction of a gas mixture, which has a cooling surface (3; 128, 228), a device for cooling the cooling surface to a temperature below the melting point of xenon, a heating device (5) for heating solid components which have condensed on the cooling device,
b) comprising an analytical device for analysing the xenon recovered, and
c) comprising a mixing device for remixing an anaesthetic gas using recovered xenon, which device sets the mixing ratio as a function of the analysis result from the analytical device.

## Revendications

1. Procédé de séparation du xénon d'un mélange gazeux provenant d'une anesthésie et comprenant les étapes suivantes :
a) on met en contact le mélange gazeux avec une surface de refroidissement mise à une température inférieure au point de fusion du xénon, sous une pression de 0,6 à 150 bars,
b) on évacue la partie du mélange gazeux qui ne se condense pas à l'état solide sur la surface de refroidissement,
c) on chauffe les composants condensés sur la surface de refroidissement au-dessus du point de fusion du xénon, cette étape de chauffage du xénon condensé sur la surface de refroidissement comprenant la liquéfaction des composants.

2. Procédé de séparation du xénon d'un mélange gazeux provenant d'une anesthésie et comprenant les étapes suivantes :
a) on met en contact le mélange gazeux avec une surface de refroidissement qui est à une température inférieure à celle du point de fusion du xénon, sous une pression de 0,6 à 150 bars,
b) on évacue la partie du mélange gazeux non condensée à l'état solide sur la surface de refroidissement,
c) on chauffe les composants condensés sur la surface de refroidissement au-delà du point de fusion du xénon, et on sépare à l'aide de deux ou plusieurs installations de séparation parallèles, et pendant l'étape de chauffage du xénon condensé dans une installation de séparation, on conduit le mélange gazeux qui arrive au moins à une autre installation de séparation pour séparer par condensation le xénon à récupérer.

3. Procédé de recyclage d'un gaz anesthésiant ayant comme composant du xénon et un ou plusieurs gaz consommables, comprenant les étapes suivantes :
a) on sépare le xénon à récupérer du mélange gazeux non nettoyé selon un procédé de séparation du xénon du mélange gazeux comprenant les étapes suivantes :
a.a) on met en contact le mélange gazeux avec une surface de refroidissement qui est à une température inférieure au point du fusion du xénon, à une pression de 0,6 à 150 bars,
a.b) on évacue la partie non condensée à l'état solide du mélange gazeux sur la surface de refroidissement,
a.c) on chauffe les composants condensés sur la surface de refroidissement au-dessus du point de fusion du xénon, le gaz consommé après séparation par condensation du xénon à récupérer étant évacué avec les impuretés,
b) on définit la teneur résiduelle de gaz consommé dans le gaz récupéré,
c) on fait un nouveau mélange du gaz avec le gaz récupéré en ajoutant une partie de gaz consommé frais, la quantité du gaz consommé ajoutée dépendant de la concentration du gaz consommé contenu encore dans le gaz récupéré.

4. Procédé selon la revendication 3,
caractérisé en ce que
le gaz consommé est de l'oxygène.

5. Application d'un dispositif comprenant :
- une installation de séparation (1 ; 120, 220) avec une enceinte pour recevoir un mélange gazeux et une surface de refroidissement (3 ; 128, 228),
- une installation pour refroidir la surface de refroidissement à une température inférieure au point de fusion du xénon,
- une installation de chauffage (5) pour chauffer les composants solides condensés sur l'installation de refroidissement,
comme faisant partie d'un appareil d'anesthésie pour séparer le xénon du mélange gazeux venant d'une anesthésie.

6. Application selon la revendication 5,
caractérisée par
un dispositif muni d'une installation pour faire passer le mélange gazeux à travers une installation de séparation (1) entre une entrée de gaz (7) avec un volume intérieur muni de surfaces de refroidissement (3) jusqu'à une sortie de gaz (8), la surface de refroidissement (3) étant située dans le chemin de circulation du gaz.

7. Application selon l'une quelconque des revendications 5 ou 6,
caractérisée en ce que
l'installation de séparation du dispositif comporte un réservoir susceptible d'être fermé, contenant la surface de refroidissement (128) et l'installation de chauffage.

8. Application selon l'une quelconque des revendications 5 à 7,
caractérisée par
un dispositif équipé d'une pompe (114) pour introduire le mélange gazeux dans le réservoir sous pression et une installation (124) pour évacuer le gaz de tête du réservoir.

9. Application selon l'une quelconque des revendications 5 à 8,
caractérisée par
un dispositif à deux ou plusieurs installations de séparation (120, 220) et une installation de commutation (116) pour fournir au choix le mélange gazeux à l'une des installations de séparation (120, 220).

10. Appareil d'anesthésie comprenant :
a) un dispositif pour récupérer du xénon provenant de gaz d'anesthésie avec une installation de séparation (1 ; 120, 220) et une enceinte pour recevoir un mélange gazeux, cette enceinte ayant une surface de refroidissement (3 ; 128, 228), une installation pour refroidir la surface de refroidissement à une température en dessous du point de fusion du xénon, une installation de chauffage (5) pour chauffer les composants solides condensés sur l'installation de refroidissement,
b) une installation d'analyse pour analyser le xénon récupéré, et
c) une installation de mélange pour mélanger de nouveau un gaz anesthésiant en utilisant le xénon récupéré, le rapport de mélange étant réglé en fonction du résultat de l'analyse fourni par l'installation d'analyse.
